(19) Europäisches Patentamt

European Patent Office

Office européen des brevets

(11) Publication number: **0 325 413**
**A2**

(12) # EUROPEAN PATENT APPLICATION

(21) Application number: **89300416.8**

(22) Date of filing: **18.01.89**

(51) Int. Cl.4: **G 01 N 33/49**
**G 01 N 33/543**
**// G01N33/52**

(30) Priority: **19.01.88 US 145090**

(43) Date of publication of application:
**26.07.89 Bulletin 89/30**

(84) Designated Contracting States:
**AT BE CH DE ES FR GB IT LI NL SE**

(71) Applicant: **SYNTEX (U.S.A.) INC.**
**3401 Hillview Avenue**
**Palo Alto, California 94304 (US)**

(72) Inventor: **Jeong, Henry**
**3396 Middlefield Road**
**Palo Alto California 94306 (US)**

**Ghazarossian, Vartan**
**340 Olive Street**
**Menlo Park California 94025 (US)**

(74) Representative: **Armitage, Ian Michael et al**
**MEWBURN ELLIS & CO. 2/3 Cursitor Street**
**London EC4A 1BQ (GB)**

(54) **Method and device for separating plasma from red cells.**

(57) Methods and devices are disclosed for separating plasma from red blood cells in a whole blood sample. The methods comprise contacting at least a portion of a bibulous support having a polycationic surface with a whole blood sample wherein the red blood cells but not plasma bind to the polycationic surface of the support and separating the plasma from the portion of the support to which the red blood cells are bound from the sample. The method has application among others to the determination of an analyte in a sample. Plasma separated from whole blood sample in accordance with the above procedure may be analyzed by any number of assay techniques. The devices that are disclosed can be used for determining an analyte in a whole blood sample and comprise a support at least a portion of which has a polycationic surface for binding red blood cells and at least a portion of which contains a specific binding pair [sbp] member non-diffusively bound thereto.

Bundesdruckerei Berlin

## Description

## METHOD AND DEVICE FOR SEPARATING PLASMA FROM RED CELLS

This invention relates to methods and devices for separating plasma from red blood cells in a whole blood sample. The invention has particular application, among others, to the determination of an analyte in whole blood.

In assays for an analyte, red blood cells may inhibit binding between specific binding pair (sbp) members. Likewise, red blood cells have enzyme activity which; depending on the assay employed, may interfere with the signal produced. Thus, assays requiring high precision are typically performed on blood serum or plasma. It is generally necessary, therefore, to separate the red blood cells from plasma in a whole blood sample suspected of containing an analyte prior to performing the assay.

Numerous techniques are known for separating red blood cells from plasma in a whole blood sample and for determining the presence or amount of an analyte in a blood sample. One commonly used method of removing plasma or serum from red blood cells is by centrifugation of whole blood. In this procedure the red blood cells settle at the bottom of the test tube and the serum is separated, for example, by decantation or some other method. Centrifugation has several disadvantages. Firstly, separation of red blood cells from a whole blood sample requires that a relatively large sample of whole blood be drawn from the patient. Further, most physician's office are not equipped with blood separation equipment. Therefore, after the whole blood sample is collected it is transferred for processing to a laboratory or other facility having such equipment. Additionally, because of the extra handling, the risk of exposure to potential hazards of blood-borne pathogens is increased.

Polybrene (hexadimethrine bromide), a quarternary ammonium polyalkyleneamine and other polycations have been used to detect red blood cell antibodies. In one such test, red blood cells are incubated with the test sera in a low ionic medium. Polybrene is introduced to cause non-specific red blood cell aggregation. The test tubes are centrifuged and the cell-free supernatant is decanted. The effect of Polybrene on the cells is neutralized and the hemagglutination results are evaluated macroscopically or microscopically.

A need exists for a method for separating plasma from red blood cells in a whole blood sample that may find use in, e.g., a method for detecting an analyte in the sample. Desirably, such method can be applied to whole blood samples of various sizes, including small samples. A need also exists for a method that does not require centrifugation.

## 2. Description of the Related Art

U.S. Patent 4,477,575 describes a process for separating plasma or serum from whole blood employing trickling blood slowly onto a layer of glass fibers and collecting the plasma or serum. The glass fibers used have diameters of 0.2μ to about 5μ and density of 0.1 to 0.5 g/cm³. The total volume of the plasma or serum which may be separated is at most 50% of the absorption volume of the glass fiber layer.

U.S. Patent 4,594,327 describes methods and compositions for detecting an analyte in whole blood wherein the whole blood sample is combined in an aqueous medium with a binding agent for red blood cells. The aqueous medium contacts a solid bibulous element to which at least one sbp member is bound and traverses the element. A border is defined that is related to the amount of analyte. The red blood cells are aggregated at an area on the element adjacent to the air-liquid interface.

U.S. Patent No. 4,401,652 discloses a process for the preparation of stroma-free hemoglobin solutions. U.S. Patent No. 4,407,961 describes an ion-exchange system and method for isolation and determination of glycosylated hemoglobin in human blood. British Patent No. 1,037,155 concerns diagnostic agents for the detection of components contained in whole blood. U.S. Patent No. 3,418,083 discloses a hydrophobic test strip for analyzing whole blood. A test strip having a reagent-containing film layer coating embedded at least preponderantly on and into only one side of a carrier layer made of a multifilar fabric or fleece is discussed in U.S. Patent No. 4,604,264.

German Offenlegungsschrift DE 3323973A1 (Trasch, et al.) discloses substrates, especially dyes, for retaining erythrocytes on filters and allowing simple recovery of plasma from whole blood. German Offenlegungsschrift DE 3441149A1 (Limbach, et al.) and DE 3508427A1 (Limbach, et al.) describe separation of whole blood on a lectin impregnated porous matrix with repeated rinsing by diluent, especially for use in clinical analysis. European Patent Publication No. 0198628A1 (Repkin, et al.) discloses a device and method for whole blood separation and analysis.

The following publications describe methods for using Polybrene to detect antibodies and/or antigens: Ferrer et al, Transfusion (1985) 25:145-148; Lalezari et al, Transfusion (1980) 20:206-211; Lee et al, Laboratory Medicine (1974) 5:47-49; Fisher, Transfusion (1983) 23:152-154; Inglis et al, Transfusion (1978) 18:84-88; Lalezari, Transfusion (1968) 8:372-380; Lalezari et al, J. Lab. & Clin. Med. (1961) 57:868-873; Dembitzer et al, Transfusion (1972) 12:94-97.

## SUMMARY OF THE INVENTION

The methods and devices of the present invention are directed to the separation of plasma from red blood cells in a whole blood sample. The method involves contacting at least a portion of a support having a polycationic surface with a whole blood sample wherein red blood cells contained in the whole blood sample but not plasma bind to the polycationic surface of the support. Optionally, the method also includes separating the support to which the red blood cells are bound from the sample. In certain embodiments, the sample traverses at least a portion of the support away from the portion contacted by the sample.

The method of the present invention has general application in securing plasma free from red blood cells and has particular application in the assay of a whole blood sample to determine an analyte. Of special interest are assays where the analyte is a member of a specific binding pair (sbp) selected from the group consisting of ligand and complementary receptor. The method involves contacting in an aqueous medium at least part of a support having a polycationic surface with a whole blood sample wherein red blood cells but not plasma bind to the polycationic surface. The plasma is thus separated from the red blood cells bound to the support, and the plasma can then be analyzed by any convenient method for the presence of the analyte.

The device of the present invention has particular application in the determination of an analyte in a whole blood sample. The device comprises a bibulous support at least a portion of which contains a polycationic polymer for binding red blood cells and at least a portion of which contains an sbp member non-diffusively bound thereto.

The invention further includes kits for conducting the method of the invention, particularly for conducting an assay for determining an analyte in a whole blood sample suspected of containing the analyte. The kit comprises in packaged combination a support at least a portion of which contains a polycationic polymer and one or more members of a signal producing system capable of producing a detectable signal in relation to the amount of analyte present in the sample.

## DESCRIPTION OF SPECIFIC EMBODIMENTS

The present invention relates to a method of separating red blood cells from the plasma of a whole blood sample. The method involves binding of red blood cells to a polycationic surface of a support. Preferably, at least a portion of the support is impregnated with a polycationic polymer. The present method involves contacting at least a portion of a bibulous support which has a polycationic surface with a whole blood sample. The red blood cells contained in the sample bind to the polycationic surface, but the plasma does not and is usually separated from the portion of the support to which the red blood cells are bound. The plasma can be removed from the support by any number of conventional separation techniques. For example, the support can be separated from the plasma or the plasma can simply be allowed to pass out of contact with the support. The plasma can be analyzed by any of a number of conventional assay techniques for the presence of an analyte.

The present invention has application generally in obtaining plasma free from red blood cells and particularly in the fields of immunoassays and blood typing. In addition, because the sample is not subject to extra handling, the risk of exposure to potential hazards of blood-borne pathogens is reduced. The present method is simple, effective and economically attractive. Additionally, where a polycationic polymer is employed, such polymer can be indefinitely stable when immobilized. Therefore, such polymer is superior to other entrapping agents, such as antibody or lectin.

The present invention also relates to a device for determining an analyte in a whole blood sample. The device includes a bibulous support at least a portion of which contains a polycationic polymer for binding red blood cells. Additionally, at least a portion of the bibulous support has a specific binding pair member (sbp) non-diffusively bound to it.

Before proceeding further with the description of the specific embodiments of the present invention, a number of terms will be defined.

Analyte--the compound or composition to be measured, the material of interest. The analyte can be a member of a specific binding pair (sbp) and may be a ligand, which is mono- or polyvalent, usually antigenic or haptenic, and is a single compound or plurality of compounds which share at least one common epitopic or determinant site. The analyte can also be a component of a particle or can become bound to a particle during an assay. Exemplary of an analyte that is a component of a particle is an antigen on the surface of a cell such as a blood group antigen (A, B, AB, O, D, etc.) or an HLA antigen. Exemplary of an analyte becoming bound to a particle during an assay is an sbp member where a complementary sbp member is bound to a particle, glycoprotein or glycolipids where a lectin is bound to a particle, antibodies where protein A is bound to a particle, and the like. The binding involved when an analyte becomes bound to a particle can be specific or non-specific, immunological or non-immunological.

The polyvalent ligand analytes will normally be poly(amino acids), i.e., polypeptides and proteins, polysaccharides, nucleic acids, and combinations thereof. Such combinations include components of bacteria, viruses, chromosomes, genes, mitochondria, nuclei, cell membranes and the like.

The precise nature of some of the analytes together with numerous examples thereof are disclosed in U.S. Patent No. 4,299,916 to Litman, et al., particularly at columns 16 to 23.

For the most part, the polyepitopic ligand analytes employed in the subject invention will have a molecular weight of at least about 5,000, more usually at least about 10,000. In the poly(amino acid) category, the poly(amino acids) of interest will generally be from about 5,000 to 5,000,000 molecular weight, more usually from about 20,000 to 1,000,000 molecular weight; among the hormones of interest, the molecular weights will usually range from about 5,000 to 60,000 molecular weight.

A wide variety of proteins may be considered as to the family of proteins having similar structural features, proteins having particular biological functions, proteins related to specific microorganisms, particularly disease causing microorganisms, etc.

The monoepitopic ligand analytes will generally be from about 100 to 2,000 molecular weight, more usually from 125 to 1,000 molecular weight. The analytes of interest include drugs, metabolites, pesticides, pollutants, and the like. Included among drugs of interest are the alkaloids. Among the alkaloids are morphine alkaloids, which includes morphine, codeine, heroin, dextromethorphan, their derivatives and metabolites; cocaine alkaloids, which include cocaine and benzoyl ecgonine, their derivatives and metabolites, ergot alkaloids, which include the diethylamide of lysergic acid; steroid alkaloids; iminazoyl alkaloids; quinazoline alkaloids, isoquinoline alkaloids; quinoline alkaloids, which include quinine and quinidine; diterpene alkaloids, their derivatives and metabolites.

The next group of drugs includes steroids, which includes the estrogens, estogens, androgens, andreocortical steroids, bile acids, cardiotonic glycosides and aglycones, which includes digoxin and digoxigenin, saponins and sapogenins, their derivatives and metabolites. Also included are the steroid mimetic substances, such as diethylstilbestrol.

The next group of drugs is lactams having from 5 to 6 annular members, which include the barbituates, e.g. phenobarbital and secobarbital, diphenylhydantonin, primidone, ethosuximide, and their metabolites.

The next group of drugs is aminoalkylbenzenes, with alkyl of from 2 to 3 carbon atoms, which includes the amphetamines, catecholamines, which includes ephedrine, L-dopa, epinephrine, narcine, papaverine, and their metabolites.

The next group of drugs is benzheterocyclics which include oxazepam, chlorpromazine, tegretol, imipramine, their derivatives and metabolites, the heterocyclic rings being azepines, diazepines and phenothiazines.

The next group of drugs is purines, which includes theophylline, caffeine, their metabolites and derivatives.

The next group of drugs includes those derived from marijuana, which includes cannabinol and tetrahydrocannabinol.

The next group of drugs includes the vitamins such as A, B, e.g. $B_{12}$, C, D, E and K, folic acid, thiamine.

The next group of drugs is prostaglandins, which differ by the degree and sites of hydroxylation and unsaturation.

The next group of drugs is antibiotics, which include penicillin, chloromycetin, actinomycetin, tetracycline, terramycin, the metabolites and derivatives.

The next group of drugs is the nucleosides and nucleotides, which include ATP, NAD, FMN, adenosine, guanosine, thymidine, and cytidine with their appropriate sugar and phosphate substituents.

The next group of drugs is miscellaneous individual drugs which include methadone, meprobamate, serotonin, meperidine, amitriptyline, nortriptyline, lidocaine, procaineamide, acetylprocaineamide, propranolol, griseofulvin, valproic acid, butyrophenones, antihistamines, anticholinergic drugs, such as atropine, their metabolites and derivatives.

Metabolites related to diseased states include spermine, galactose, phenylpyruvic acid, and porphyrin Type 1.

The next group of drugs is aminoglycosides, such as gentamicin, kanamicin, tobramycin, and amikacin.

Among pesticides of interest are polyhalogenated biphenyls, phosphate esters, thiophosphates, carbamates, polyhalogenated sulfenamides, their metabolites and derivatives.

For receptor analytes, the molecular weights will generally range from 10,000 to $2 \times 10^8$, more usually from 10,000 to $10^6$. For immunoglobulins, IgA, IgG, IgE and IgM, the molecular weights will generally vary from about 160,000 to about $10^6$. Enzymes will normally range from about 10,000 to 1,000,000 in molecular weight. Natural receptors vary widely, generally being at least about 25,000 molecular weight and may be $10^6$ or higher molecular weight, including such materials as avidin, DNA, RNA, thyroxine binding globulin, thyroxine binding prealbumin, transcortin, etc.

Ligand analog or analyte analog--a modified ligand or ligand surrogate or modified analyte or analyte surrogate which can compete with the analogous ligand or analyte for a receptor, the modification providing means to join a ligand analog or analyte analog to another molecule. The ligand analog or analyte analog will usually differ from the ligand or analyte by more than replacement of a hydrogen with a bond which links the ligand analog or analyte analog to a hub or label, but need not. The term ligand surrogate or analyte surrogate refers to a compound having the capability of specifically binding a receptor complementary to the ligand or analyte. Thus, the ligand surrogate or analyte surrogate can bind to the receptor in a manner similar to the ligand or analyte. The surrogate could be, for example, an antibody directed against the idiotype of an antibody to the ligand or analyte.

Poly(ligand analog)--a plurality of ligand analogs joined together covalently, normally to a hub nucleus. The hub nucleus is a polyfunctional material, normally polymeric, usually having a plurality of functional groups, e.g., hydroxyl, amino, mercapto, ethylenic, etc. as sites for linking. The hub nucleus may be water soluble or insoluble, preferably water soluble, and will normally be at least about 30,000 molecular weight and may be 10 million or more molecular weight. Illustrative hub nuclei include polysaccharides, polypeptides (including proteins), nucleic acids, anion exchange resins, and the like. Water insoluble bub nuclei can also include walls of containers, e.g. glass or plastic, glass beads, addition and condensation polymers, Sephadex and Agarose beads and the like.

Member of a specific binding pair ("sbp member")--one of two different molecules, having an area on the surface or in a cavity which specifically binds to and is thereby defined as complementary with a particular spatial and polar organization of the other molecule. The members of the specific binding pair are referred to as ligand and receptor (antiligand). These will usually be members of an immunological pair such as antigen-antibody, although other specific binding pairs such as biotin-avidin, hormones-hormone receptors, nucleic acid duplexes, IgG-protein A, DNA-DNA, DNA-RNA, and the like are not immunological pairs but are included in the invention.

Ligand--any organic compound for which a receptor naturally exists or can be prepared.

Receptor ("antiligand")--any compound or composition capable of recognizing a particular spatial and polar organization of a molecule, e.g., epitopic or determinant site. Illustrative receptors include naturally occurring receptors, e.g., thyroxine binding globulin, antibodies, enzymes, Fab fragments, lectins, nucleic acids, protein A, complement component Clq, and the like.

Label--A member of the signal producing system that is conjugated to an sbp member. The label can be isotopic or non-isotopic, usually non-isotopic, including catalysts such as an enzyme, a chromogen such as a fluorescer, dye or chemiluminescer, a radioactive substance, a particle, and so forth.

·Signal Producing System--The signal producing system may have one or more components, at least one component being a label. The signal producing system generates a signal that relates to the presence or amount of analyte in a sample. The signal producing system includes all of the reagents required to produce a measurable signal. When the label is not conjugated to an sbp member analogous to the analyte, the label is normally bound to an sbp member complementary to an sbp member that is analogous to the analyte. Other components of the signal producing system can include substrates, enhancers, activators, chemiluminiscent compounds, cofactors, inhibitors, scavengers, metal ions, specific binding substances required for binding of signal generating substances, and the like. Other components of the signal producing system may also be coenzymes, substances that react with enzymic products, other enzymes and catalysts, and the like. The signal producing system provides a signal detectable by external means, preferably by measurement of the degree of aggregation of particles or by use of electromagnetic radiation, desirably by visual examination. For the most part, the signal producing system will involve particles, such as fluorescent particles or other light absorbing particles, a chromophoric substrate and enzyme, where chromophoric substrates are enzymatically converted to dyes which absorb light in the ultraviolet or visible region, phosphors, fluorescers or chemiluminescers.

The signal-producing system can include at least one catalyst, usually an enzyme, and at least one substrate and may include two or more catalysts and a plurality of substrates, and may include a combination of enzymes, where the substrate of one enzyme is the product of the other enzyme. The operation of the signal producing system is to produce a product which provides a detectable signal related to the amount of analyte in the sample.

A large number of enzymes and coenzymes useful in a signal producing system are indicated in U.S. Patent No. 4,275,149, columns 19 to 23, and U.S. Patent No. 4,318,980, columns 10 to 14. A number of enzyme combinations are set forth in U.S. Patent No. 4,275,149, columns 23 to 28, which combinations can find use in the subject invention.

Of particular interest are enzymes which involve the production of hydrogen peroxide and the use of the hydrogen peroxide to oxidize a dye precursor to a dye. Particular combinations include saccharide oxidases, e.g., glucose and galactose oxidase, or heterocyclic oxidases, such as uricase and xanthine oxidase, coupled with an enzyme which employs the hydrogen peroxide to oxidize a dye precursor, that is, a peroxidase such as horse radish peroxidase, lactoperoxidase, or microperoxidase. Additional enzyme combinations may be found in the subject matter incorporated by reference. When a single enzyme is used as a label, other enzymes may find use such as hydrolases, transferases, and oxidoreductases, preferably hydrolases such as alkaline phosphatase and β-galactosidase. Alternatively, luciferases may be used such as firefly luciferase and bacterial luciferase.

Illustrative coenzymes which find use include NAD[H]; NADP[H], pyridoxal phosphate; FAD[H]; FMN[H], etc., usually coenzymes involving cycling reactions, see particularly U.S. Patent No. 4,318,980.

The product of the enzyme reaction will usually be a dye chemiluminescer or fluorescer. A large number of illustrative fluorescers are indicated in U.S. Patent No. 4,275,149, columns 30 and 31.

Polycationic surface - a surface of a support having a plurality of positive charges, usually at least $10^{11}$ positive charges per $\mu m^2$, usually $10^{12}$ to $10^{15}$ per $\mu m^2$. The polycationic surface can be achieved by virtue of containing a polycationic reagent. On the other hand, the support can be treated directly to induce positive charges such as by treatment of paper with cyanogen bromide followed by ethylene diamine or treatment of nylon with triethyl oxonium fluroburates or treatment of glass with alumoethyl trimethoxy silane.

Polycationic reagent--a compound, composition, or material, usually polymeric, either inorganic or organic, naturally occurring or synthetic, having at least two positive charges, preferably at least ten positive charges; e.g., a polyelectrolyte, usually 20 to 1000 positive charges.

Exemplary of polycationic reagents are polyalkylene amines (wherein the alkylene has 1 to 20 carbon atoms, preferably 2 to 4 carbon atoms) such as polyethyleneimine and polypropyleneimine and their lower alkyl (1 to 6 carbon,atoms) ammonium salts such as Polybrene, 1,5-dimethyl- 1,5-diazaundecamethylene polymethobromide, or metal ions such as calcium and barium ion, aminodextrans, protamine, chitosan, positively charged liposomes, polylysine, and the like. Polycationic polymers also include pseudo-brene and hydroxybrene disclosed in copending U.S. application Serial No. 051,978, filed May 19, 1987 (corresponding to Canadian Patent Application S.N. 567176, Japanese Patent Application No. 63-121680, or British Patent Application No. 8811751 (Publication No. 2206206).

Other methods of applying positive charge to the surface involve chemical linking of e.g., ammonium sulfonium and phosphonium via ester, ether, carbonate, sulfonate linkers and the like.

Support--a porous water insoluble material. The support can be hydrophilic or capable of being rendered hydrophilic and includes inorganic powders such as silica, magnesium sulfate, and alumina; natural polymeric materials, particularly cellulosic materials and materials derived from cellulose, such as fiber containing papers, e.g., filter paper, chromatographic paper, etc.; synthetic or modified naturally occurring polymers, such as nitrocellulose, cellulose acetate, poly(vinyl chloride), polyacrylamide, cross linked dextran, agarose, polyacrylate, polyethylene, polypropylene, poly(4-methylbutene), polystyrene, polymethacrylate, poly(ethylene terephthalate), nylon, poly(vinyl butyrate), etc.; either used by themselves or in conjunction with other materials; glass, ceramics, metals, and the like.

Bibulous support--any bibulous absorbent solid material which allows for binding of a polycationic polymer directly or indirectly to its surface and allows for the transport of the components of a whole blood sample, for example by capillary action, filtration, wicking or the like. The bibulous support is porous such that the pore size is large enough to permit the passage of red blood cells in those instances where the support does not have a polycationic surface. The bibulous support can be paper, cellulose particles, silica gel, cellulosic beads, and the like.

The size and shape of the bibulous support may be varied widely and will depend on whether the support is utilized in a filter mode or in a mode wherein wicking or capillary flow is the primary result. Consideration should be given to the purpose of the support, namely, to retain the bound red blood cells and, where applicable, to be a support on which to conduct an assay for an analyte. In individual separations and/or assays, for example, where filtration or wicking is chosen as the mode for separation or is utilized in the assay, one size and/or shape may be preferred over another depending, e.g., on the amount of whole blood subjected to the present method. The support may be shaped, by way of example but not of limitation, as a strip, triangular, rectangular or square-shaped structure, a circular or oval disc or the like. Exemplary of such supports are those disclosed in U.S. Patent Nos, 4,552,839; 4,366,241; 4,594,327; and 4,235,601.

The surface of the support can have different physical characteristics and can be of different chemical compositions and may be of one or more compositions such as a mixture of compositions or laminates or combinations thereof. Various materials may be employed, taking into consideration the ease of introducing a polycationic surface such as by immobilizing a polycationic polymer on the surface of the support and the ease of non-diffusively binding an sbp member to the surface of the support when the support will be used to conduct an assay for an analyte.

A polycationic reagent can be placed on a support by convalent or non-convalent binding. In one embodiment a polycationic polymer is immobilized on the support by ionic interactions with the support at the site of application. By way of example, but not of limitation, an anionic support such as paper allows a polycationic polymer such as Polybrene to be immobilized on it by virture of charge-charge interactions. The polycationic polymer may be applied to a portion of a support, e.g. one end of a paper strip. Alternatively, the polymer may be applied over the entire support. After application of the polycationic polymer to the support, the support is dried, for example, by evaporating the polymeric liquid, for example under a stream of nitrogen.

In another embodiment the polycationic polymer is immobilized on the support by employing a linking agent. In such a case, the polymer generally has, or will be treated to have, an amine or acid group and the support has a corresponding acid or amine group as the case may be. Examples of such linking agents include carbodiimides such as ethyl dimethylaminopropyl carbodiimide, dicyclohexyl-carbodiimide and the like.

Bibulous support - sbp member conjugate (immunochromatograph)--at least one sbp member is non-diffusively bound to the surface of the bibulous support, either covalently or non-covalently. This may be accomplished according to the techniques described, for example, in U.S. Pat. Nos. 4,168,146 and 4,299,916.

The binding of the sbp member to the surface is in a region which allows for the movement of a liquid across the region with transport of the analyte and, as appropriate, any members of the signal producing system. One or more members of the signal producing system may be non-diffusively bound to the bibulous support, either covalently or non-covalently.

Ancillary Materials--Various ancillary materials will frequently be employed in an assay in accordance with the present invention. For example, buffers will normally be present in the assay medium, as well as stabilizers for the assay medium and the assay components. Frequently, in addition to these additives, additional proteins may be included, such as albumins, or surfactants, particularly non-ionic surfactants, binding enhancers, e.g., polyalkylene glycols, or the like.

As mentioned above, the present invention involves a method for separating plasma from red blood cells in a whole blood sample. The method comprises a solid-phase separation of plasma from red blood cells in whole blood utilizing a support having a polycationic surface. When a whole blood sample is introduced on to the support non-specific binding between the polycationic surface and the red blood cells occurs and the cells are trapped on the polycationic surface. Red blood cells generally have a negative charge by virtue of sialic acid residues or the like on the surface of the cells. Plasma, on the other hand, does not bind to, and moves away from, the polycationic surface. In this way the plasma is separated from the red blood cells. Generally, the plasma is removed from the polycationic surface by wicking along the bibulous support away from the polycationic surface due to capillary flow or by passing through the bibulous support as the result of filtration.

The polycationic reagent is generally employed in amounts sufficient to achieve binding of essentially all of the red blood cells when a whole blood sample is contacted with a portion of the support. Generally, the amount of polycationic reagent required to be bound to the support or the size of the polycationic surface or both increase as the size of the sample to be treated increases. Additionally, the nature and size of the support and the nature of the polycationic reagent affect the amount of polycationic reagent contained on an individual support. Generally, a support contains at least 100 positively charged groups per cell that becomes bound, frequently at least 1000, more frequently over 10,000, Usually cells will be caused to bind to bibulous material of a 1 mm² to 500 mm², frequently 10 mm² to 100 mm² nominal area.

In carrying out the separation methods of the present invention, an aqueous medium will be used. Other polar solvents may be included, usually oxygenated organic solvents of from 1-6, more usually 1-4 carbon atoms, including alcohols, ethers and the like. Usually these cosolvents will be present in less than about 40 weight percent, more usually less than 20 weight percent.

Normally the separation methods of the present invention are carried out at a moderate pH to maximize separation of red blood cells and plasma from whole blood. The pH for the medium will generally be in the range of about 4-11, more usually in the range of 6-10. Various buffers may be employed to achieve the desired pH and maintain the pH during the separation. Illustrative buffers include borate, phosphate, carbonate, TRIS, barbital and the like. The particular buffer is not critical to this invention but in individual assays, one buffer may be preferred over another.

The ionic strength will generally vary from 0.05 to 0.5 preferably about 0.1 to 0.3. The pH and the ionic strength may be optimized within the above ranges depending on the particular assay involved. Usually low ionic strength buffers will be used without organic solvents.

Moderate temperatures are generally employed during the separation. Temperature during separation and measurements will generally range from about 10° to 50°C, more usually from about 15° to 40°C.

It is an advantage of the present method that after the whole blood sample contacts the polycationic polymer, the red blood cells are rapidly separated from the sample. The separation will generally take from about 0.5 minutes to about 10 minutes, more usually from about 1 minute to 5 minutes.

As mentioned above, the invention involving separating of plasma from red cells can be practiced in a number of ways. Usually, the particular mode of carrying out the separation is determined by the purpose of the separation. For preparation of both small and large quantities (1μl to several liters or more) of plasma free from red blood cells, a column or filtration mode will normally be employed. In a column mode the support (e.g., beads) having a portion with a polycationic surface is placed in the column. The whole blood is then allowed to pass through the column by gravitational flow, positive pressure, or the like. The red blood cells bind to the polycationic surface while the plasma exits the column. In a filtration mode the support having a polycationic surface is in the form of a filter that will allow plasma to pass therethrough while retaining the red blood cells on the polycationic surface. The so-separated plasma is collected in an appropriate container.

Small amounts (500 μl or less) of plasma can be separated from red blood cells in accordance with the invention by employing a support having a polycationic surface portion in a wicking mode. The whole blood sample is applied to the support in manner that allows the red blood cells to bind to the polycationic surface and the plasma to be transported away from such surface by capillary migration. Red blood cells can be released from the support, if desired, by interfering with the charge-charge interactions which cause the binding of the cells to the support. Such release can be obtained by contacting the support having bound red cells with an aqueous medium, usually buffered, having an ionic strength sufficient to disrupt the binding but insufficient to cause damage to the red blood cells, preferably an ionic strength of from about 0.1 to 0.6. A specific example for the release of the bound red blood cells is the use of a polyanion, for example, polyacrylic acid, M.W. 5,000, in an aqueous medium, usually buffered, at molar concentrations from about 0.5 mM to 20 mM. Other methods for separating red blood cells from the support will be suggested to those skilled in the art.

The present method for separation of plasma from red blood cells in a whole blood sample has application to assay methods for the determination of an analyte in a whole blood sample suspected of containing the analyte. The assay method of the present invention comprises, prior to conducting an assay method, contacting at least a part of a support at least a portion of which has a polycationic surface with a whole blood sample to bind the red blood cells but not the plasma.

Plasma obtained by the separation method of the present invention may be assayed for an analyte suspected of being in the sample by any of a number of assay methodologies. The assays may be heterogeneous or homogeneous, quantitative, semi-quantitative or qualitative.

Exemplary of heterogeneous assays are the radioimmunoassay (RIA, Yalow and Berson, J. Clin. Invest. (1960) 39, 1157), and enzyme linked immunoassays such as the enzyme linked immunosorbant assay (ELISA), see "Enzyme-Immunoassay" by Edward T. Maggio, CRC Press Incorporated, Boca Raton, Florida, 1980. Homogeneous immunoassays are exemplified by enzyme multiplied immunoassay techniques (e.g. see U.S. Patent No. 3,817,837), immunofluorescence methods such as those disclosed in U.S. Patent No. 3,993,345, enzyme channeling techniques such as those disclosed in U.S. Patent No. 4,233,402, and other enzyme immunoassays as discussed in Maggio, supra.

The present invention may be used with assays involving test strips such as those disclosed in U.S. Patent Nos. 4,594,327; 4,168,146; and 4,366,241; or test cards such as 3,990,850; and 4,055,394.

The assay for the analyte will generally be carried out under the same conditions as those used in the separation of plasma from red blood cells in accordance with the present invention. Where plasma is separated from the red blood cells and then subsequently subjected to an assay method, the condition for the assay may or may not be the same as those for the separation. Generally, an aqueous buffered medium at a moderate pH is employed, which provides optimum assay sensitivity.

The aqueous medium may be solely water or may include from 0 to 40 volume percent of a cosolvent. The pH for the medium will usually be in the range of about 4 to 11, more usually in the range of about 5 to 10, and preferably in the range of about 6.5 to 9.5. The pH will usually be a compromise between optimum binding of the binding members and the pH optimum for other reagents of the assay such as members of the signal producing system.

Various buffers may be used to achieve the desired pH and maintain the pH during the determination. Illustrative buffers include borate, phosphate, carbonate, tris, barbital and the like. The particular buffer employed is not critical to this invention, but in an individual assay one or another buffer may be preferred.

Moderate temperatures are normally employed for carrying out the assay and usually constant temperatures during the period of the measurement, particularly for rate determinations. Incubation temperatures will normally range from about 5° to 45°C, more usually from about 15° to 40°C. Temperatures during measurements will generally range from about 10° to 50°, more usually from about 15° to 40°C.

The concentration of analyte which may be assayed will generally vary from about $10^{-4}$ to $10^{-15}$ M, more usually from about $10^{-6}$ to $10^{-14}$ M. Considerations, such as whether the assay is qualitative, semiquantitative or quantitative, the particular detection technique and the concentration of the analyte of interest will normally determine the concentrations of the various reagents.

While the concentrations of the various reagents in the assay medium will generally be determined by the concentration range of interest of the analyte, the final concentration of each of the reagents will normally be determined empirically to optimize the sensitivity of the assay over the range. That is, a variation in concentration of the analyte which is of significance should provide an accurately measurable signal difference.

While the order of addition may be varied widely, there will be certain preferences depending on the nature of the assay, the particular labels, the compound to which the label is conjugated, the nature of the conjugates, the nature of the analyte, and the relative concentrations of the analyte and reagents.

In one embodiment, the present invention can be applied to enzyme immunochromatographic methods including those disclosed in U.S. Patent Nos. 4,168,146; 4,435,504; and 4,594,327. For example, an analyte in a whole blood sample is determined by contacting in an aqueous medium a region of a bibulous support, at least a portion of which has a polycationic surface, with the whole blood sample suspected of containing the analyte. Where the analyte is a member of a specific binding pair, at least a portion of the support may contain an sbp member non-diffusively bound to it. Conditions are selected which allow the red blood cells to bind to the polycationic surface and to permit the medium containing the plasma to traverse at least a portion of the support to define a border related to the amount of analyte in the whole blood sample. Thereafter, the border is determined. Where appropriate to the assay protocol, the border is determined by including in the assay medium, or in a subsequent medium, one or more members of a signal producing system, which includes a labeled sbp member and is capable of generating a signal in relation to the amount of analyte in the sample.

The labeled sbp member may be present in the aqueous medium containing the whole blood sample prior to contact with the support. The labeled spb member may be present in a reagent solution or developer solution used after the aqueous medium has contacted the support or the labeled sbp member may be bound to the support. In the first approach either the sbp member-label contacts the support concurrently with the analyte, so as to actually compete with the analyte for available binding sites; or, the sbp member-label does not have an apparent competition and primarily binds in a zone immediately beyond the zone in which the analyte has bound.

Where the sbp member is added after the sample has contacted the support and the analyte is antigenic, the support is immersed in a solution containing labeled sbp member, which binds to the antigen. In those cases involving a hapten, one can provide for a fixed amount of a polyligand, that is, the ligand can be polymerized or conjugated to a hub nucleus, so as to provide for a plurality of determinant sites common to both the haptenic analyte and the polyligand. In effect, an antigen is produced where the extent of travel of the synthetic antigen will be related to the amount of analyte in the sample.

Where the assay is not standardized to the extent that variations in conditions may change the distance the analyte traverses, a standard sample can be provided having a known amount of analyte. The analyte sample and the standard can be run at the same time, and a quantitative comparison can be made between the standard sample and the analyte sample. If necessary, more than one standard can be employed, so that the distance traversed can be graphed for the different concentrations and used to quantitate a particular sample.

For the most part, relatively short times are involved for the assay. Where a sample traverses up or across a strip, it will take at least 30 seconds and not more than 1 hour, more usually from about 1 minute to 30 minutes. The development of the signal will generally range from 30 seconds to 30 minutes, more usually from about 30 seconds to 5 minutes.

If the label-sbp member conjugate is not bound to the support and was not combined with the sample, the immunochromatograph is contacted substantially uniformly with a developer solution having labeled-sbp member conjugate after the sample has traversed the support. Depending on the label and the protocol one or more other members of the signal producing system may also be included.

The support is contacted with the developer solution for a sufficient time to produce sufficient detectable signal producing compound so as to define the region of the support in which the analyte is bound. Once the detectable signal has been produced, the distance from one end of the support may be measured as a quantitative measure of the amount of analyte in the sample.

Another example of an assay in which the present invention may be employed is described in U.S. Patent No. 4,740,468. The method and device disclosed therein are for determining the presence of an analyte in a sample suspected of containing the analyte. The method involves contacting a test solution containing the sample and the first member of a specific binding pair with an end portion of a test strip of bibulous material capable of being transversed by the test solution through capillary action. The first member of the specific binding pair is capable of binding the analyte. The strip contains a second member of a specific binding pair integral therewith for concentrating and non-diffusively binding the first specific binding pair member at a small situs on the strip separated from the end portion of the strip. A detectible signal is produced in relation to the presence of the analyte in the test solution.

Applying the present invention to the method and device, a first small situs close to a first end can contain the polycationic reagent in accordance with the present invention and a second situs further up the strip can contain the second member of the specific binding pair. The sample suspected of containing the analyte and binding partner for such analyte are combined in an aqueous medium. The medium is contacted with the first end portion of the strip and allowed to migrate along the strip by capillary action. The red blood cells are separated from the sample upon contact with the polycationic reagent on the support. If analyte is present in the sample, an immune complex will form between the analyte and its cognate binding partner. This immune complex will then be captured at the second situs. Members of a signal producing system are employed to generate a signal at the second situs as a result of the captured immune complex. On the other hand, if analyte is not present in the sample no immune complex will form and be captured when the aqueous medium containing the sample and the antibody for the analyte is contacted with the end portion of the strip and allowed to migrate along the strip by capillary action to the situs. Therefore, no signal will be produced at the second situs thereby indicating the absence of analyte in the sample.

To enhance the versatility of the present invention, the device of the present invention can be provided in a kit in packaged combination with a support at least a portion of which contains a polycationic surface and one or more members of a signal producing system capable of producing a detectable signal in relation to the amount of analyte present in the sample. The support may also include an sbp member non-diffusively bound thereto. The kit further includes other reagents for conducting an assay including members of the signal producing system each packaged alone or in combination with one or more of each other depending on the compatibility of such reagents.

## EXAMPLES

The invention is demonstrated further by the following illustrative examples.

Before proceeding with a description of the Examples, the following terms are defined:

| | |
|---|---|
| Polybrene - | 1,5-dimethyl-1,5-diazaundecamethylene polymethobromide (Aldrich Chemical Co.) |
| LISS - | (Low ionic salt solution buffer) 0.024M glycine; 0.03M NaCl; 0.0017M $H_2PO_4$; 0.0013M $Na_2PO_4$; 0.1% $NaN_3$; |
| Paper - | Whatman 31 ET Chrom |
| EDTA - | Ethylenediaminetetraacetic acid |

## EXAMPLE 1

Approximately 50 µl of Polybrene (100 mg/ml) in LISS was applied across one end (0.5 cm from bottom edge) of a paper strip (0.5 x 5 cm) and the liquid was evaporated under a stream of nitrogen gas, 10 µl of blood (EDTA as anticoagulant) was diluted in 1 ml of LISS. The strip was placed in a tube containing the diluted blood and the solution was allowed to wick by capillary action. A distinct red band indicating red blood cell entrapment was observed at the site of the Polybrene application. Plasma wicked past the red band to the top of the strip. The control strip (no polybrene) exhibited a red smear, indicating no entrapment of red blood cells.

Although the foregoing invention has been described by way of illustration and example for purposes of clarity and understanding, it will be obvious that certain modifications and changes may be practiced within the scope of the appended claims.

**Claims**

1. A method for separating plasma from red blood cells in a whole blood sample, which comprises contacting at least a portion of a bibulous support having a polycationic surface with a whole blood sample wherein red blood cells but not plasma contained in said whole blood sample bind to said polycationic surface.

2. The method of Claim 1 wherein said support contains a polycationic reagent, preferably wherein said polycationic reagent is selected from the group consisting of polybrene, chitosan, polyalkyleneimine, pseudo-brene, polylysine and hydroxybrene.

3. The method of claim 1 or claim 2 which further comprises determining an analyte in a whole blood sample by analyzing plasma of said sample for the presence of an analyte.

4. The method of any one of claims 1 to 3 wherein said plasma traverses at least a portion of said support away from said portion contacted by said sample, and wherein a border is defined related to the amount of analyte in said sample.

5. The method of Claim 4 wherein said border is determined by means of a signal producing system capable of generating a signal in relation to the amount of analyte in said sample.

6. A method for determining an analyte in a whole blood sample, said analyte being a member of a specific binding pair (sbp) selected from the group consisting of ligand and complementary receptor, which method comprises:

contacting a portion of a bibulous support containing a polycationic polymer with a whole blood sample in an aqueous medium under conditions sufficient to bind red blood cells to said polycationic polymer on said support and for allowing said medium to traverse at least a portion of said support to define a border related to the amount of analyte in said sample; and

determining said border.

7. The method of Claim 6 wherein said border is determined by means of a signal producing system capable of generating a signal in relation to the amount of analyte in said sample, which system includes a labeled sbp member.

8. A device for use in determining an analyte in a whole blood sample, said device comprising a bibulous support at least a portion of which has a polycationic surface for binding red blood cells and at least a portion of which contains an sbp member non-diffusively bound thereto.

9. The device of Claim 8 wherein said support contains a polycationic polymer selected from the group consisting of polybrene, chitosan, polyalkyleneimine, polylysine, pseudo-brene and hydroxybrene.

10. A kit for use in a method for determining an analyte in a whole blood sample, said kit comprising in packaged combination:

(a) a support at least a portion of which has a polycationic surface, and

(b) one or more members of a signal producing system capable of producing a detectable signal in relation to the amount of analyte present in the sample.